# EUROPEAN PATENT APPLICATION

(11) **EP 4 109 399 A1**
(43) Date of publication of application: **28.12.2022**
(21) Application number: 20919513.0
(22) Date of filing: 10.09.2020
(51) Int. Cl.: G06T 7/20, A61B 5/107, A61B 5/11

(54) **INFORMATION PROCESSING DEVICE, AND DETERMINATION RESULT OUTPUT METHOD**

(30) Priority: 21.02.2020 JP 2020027693
(71) Applicant: Hitachi High-Tech Corporation, Minato-ku Tokyo 105-6409 (JP)
(72) Inventor: TOYOMURA, Takashi, Tokyo 100-8280 (JP); TANAKA, Takeshi, Tokyo 100-8280 (JP); FUKUI, Daisuke, Tokyo 100-8280 (JP); NAKAGAWA, Hiromitsu, Tokyo 100-8280 (JP)
(74) Representative: MERH-IP Matias Erny Reichl Hoffmann Patentanwälte PartG mbB
(86) International application number: PCT/JP2020/034224
(87) International publication number: WO 2021/166294

(57) **Abstract**

In a motion analysis apparatus 101, a data input unit 205 acquires a first imaging result and a second imaging result. In the motion analysis apparatus 101, a skeleton recognition unit 206 recognizes skeleton positions of a subject using the first imaging result acquired by the data input unit 205, and recognizes skeleton positions of the subject using the second imaging result acquired by the data input unit 205. A motion period extraction unit 403 extracts a period from a start of a motion to an end of the motion as a range of data for comparing skeleton feature points recognized by the skeleton recognition unit 206. The similarity calculation unit 401 compares skeleton feature points recognized for an input from a depth camera with skeleton feature points recognized for an input from an RGB camera to calculate similarities, and outputs a determination result based on the similarities.

## Description

### Technical Field

The present invention relates to an information processing apparatus and a determination result output method.

### Background Art

An average life of Japanese people continues to grow and exceeds 80 years for both men and women, and a health life indicating a period in which there is no limitation in daily life is 72 years old for men and 75 years old for women, which are different from the average life.

This is because an elderly person is in a nursing-care-required state such as bedridden. A most frequent cause of a need for nursing care is movement organ disorder. A state in which a movement function is deteriorated due to the movement organ disorder is called locomotive syndrome, and in recent years, attention has been paid to prevention of the locomotive syndrome.

It is known that a sign of the locomotive syndrome appears in walking, and an expert such as a doctor diagnoses the walking of a patient from various angles.

Meanwhile, with progress of wearable acceleration sensors and improvements in camera image analysis technology, an environment in which a motion of a person can be sensed and digitally analyzed has been established. With regard to walking analysis, attempts have been made to improve an efficiency of the diagnosis performed by the expert spending time in the related art by quantifying a walking pattern by attaching the acceleration sensor to hands or feet, or by tracking behaviors of the hands or feet by camera image recognition.

For example, PTL 1 discloses a method of evaluating suitability of swings by skeleton-recognizing a motion such as golf swing using a depth camera such as "Kinect (registered trademark)" and comparing an index extracted from movements of skeleton feature points with reference data.

In addition, there is disclosed a method in which a walking identification model is constructed in advance based on three-dimensional skeleton coordinates, and conversion is added such that two-dimensional skeleton coordinates captured at any angle can be input at a time of identification.

PTL 2 discloses an apparatus related to key point data capable of obtaining information related to a predetermined posture in a three-dimensional space even when the key point data such as skeleton joint data is given as two-dimensional data.

### Citation List

### Patent Literature

PTL 1: JP-A-2014-188146
PTL 2: JP-A-2019-96113

### Summary of Invention

### Technical Problem

In recent years, as a sensing device has been commoditized, there has been an increasing need for flexibly constructing a system with a low-cost device having the same function as the acceleration sensor or a camera for cost reduction.

In PTL 2, when a device is changed, conversion is performed so as to be close to an original condition, and thus the device can be used as an input as it is, but when an input condition is changed, it is originally necessary to reconstruct the model, and the method of PTL 2 has a limit in identification accuracy.

That is, in order to ensure sufficient accuracy, it is necessary to analyze new input data and reconstruct the model, and thus there is a problem that data analysis cannot be efficiently performed.

Therefore, an object of the invention is to enable efficient data analysis.

### Solution to Problem

An information processing apparatus for processing information acquired from a device capable of capturing an image of a user according to a representative embodiment of the invention includes: a first imaging result acquisition unit configured to acquire a first imaging result that is a result of imaging performed by a first device configured to capture an image of a motion state of the user; a second imaging result acquisition unit configured to acquire a second imaging result that is a result of imaging performed by a second device configured to capture an image of the motion state of the user; a skeleton recognition unit configured to recognize a skeleton position of the user by using the first imaging result acquired by the first imaging result acquisition unit and recognize a skeleton position of the user by using the second imaging result acquired by the second imaging result acquisition unit; a motion period specifying unit configured to specify a predetermined motion period of the user based on a result obtained by the recognition performed by the skeleton recognition unit; a similarity calculation unit configured to calculate a similarity between a change in the skeleton position of the user recognized by the skeleton recognition unit using the first imaging result and a change in the skeleton position of the user recognized by the skeleton recognition unit using the second imaging result in the motion period specified by the motion period specifying unit; and a determination result output unit configured to output a determination result based on the similarity calculated by the similarity calculation unit.

### Advantageous Effects of Invention

According to the invention, data analysis can be efficiently performed.

### Brief Description of Drawings

[FIG. 1] FIG. 1 is a diagram showing an outline of a system configuration including a motion analysis apparatus.
[FIG. 2] FIG. 2 is a diagram showing an example of a configuration of the motion analysis apparatus according to the present embodiment.
[FIG. 3] FIG. 3 is a schematic diagram of a system including the motion analysis apparatus that compares imaging results obtained by a plurality of skeleton measurement units.
[FIG. 4] FIG. 4 is a diagram showing an example of the configuration of the motion analysis apparatus according to the present embodiment.
[FIG. 5] FIG. 5 is a diagram showing an example of an input screen for inputting a correspondence relationship between skeleton feature points.
[FIG. 6] FIG. 6 is a flowchart showing a processing procedure for calculating a similarity of the skeleton feature points in a similarity calculation unit.
[FIG. 7] FIG. 7 is a diagram showing an example of a screen configuration for presenting the similarity to a user.
[FIG. 8] FIG. 8 is a diagram showing an example of a screen example showing a list of correspondences between the skeleton feature points and the similarities.
[FIG. 9] FIG. 9 is a diagram showing a configuration of a system that performs measurement using a motion analysis apparatus according to a second embodiment.
[FIG. 10] FIG. 10 is a diagram showing an example of a configuration of the motion analysis apparatus according to the present embodiment according to a second embodiment.
[FIG. 11] FIG. 11 is a diagram showing an input screen of walking types.
[FIG. 12] FIG. 12 is a diagram showing an example of a screen of a determination result.
[FIG. 13] FIG. 13 is a flowchart in which the motion analysis apparatus estimates a correspondence relationship between skeleton feature points of a first measurement unit and a second measurement unit, and generates a skeleton correspondence table.
[FIG. 14] FIG. 14 is a diagram showing a configuration of a motion analysis apparatus according to a fourth embodiment.
[FIG. 15] FIG. 15 is a flowchart of specifying similar skeleton feature points of a first measurement unit with respect to skeleton feature points (target feature points) of the first measurement unit which do not exist in a second measurement unit.

### Description of Embodiments

Hereinafter, embodiments of the invention will be described with reference to the drawings.

### (First Embodiment)

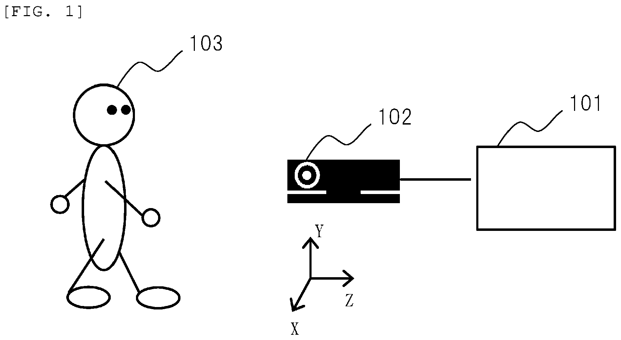

FIG. 1 is a diagram showing an outline of a system configuration including a motion analysis apparatus 101 according to the present embodiment. The motion analysis apparatus 101 is an information processing apparatus that processes information acquired from a device (for example, a depth camera 102), and is a server apparatus or the like. The motion analysis apparatus 101 is connected to the depth camera 102 (first device) by a USB cable or the like. Accordingly, the motion analysis apparatus 101 can acquire the information from the depth camera 102.

The motion analysis apparatus 101 may transmit and receive information to and from the depth camera 102 via a wireless or wired network.

The depth camera 102 is a known depth camera, generates information (depth information) in an X-axis direction, a Y-axis direction, and a Z-axis direction of an imaging target as a result of imaging, and stores the depth information.

The depth camera 102 can capture an image of a state in which a subject 103 (user) is walking (for example, a state in which the subject 103 is walking toward the depth camera 102). That is, the depth camera 102 is a device capable of capturing an image of the user, and is a device capable of capturing an image of a motion state of the user.

The motion analysis apparatus 101 is an apparatus that acquires information from a device (such as the depth camera 102) capable of capturing an image of the user and processes the information. Specifically, the motion analysis apparatus 101 acquires the depth information based on the result of imaging the state in which the subject 103 is walking. The motion analysis apparatus 101 specifies skeleton positions (for example, a skeleton position of a left elbow) of the subject 103 from the depth information, specifies changes in the specified skeleton positions, and determines whether the subject 103 is in locomotive syndrome based on the changes. In this way, the motion analysis apparatus 101 measures how the subject 103 walks.

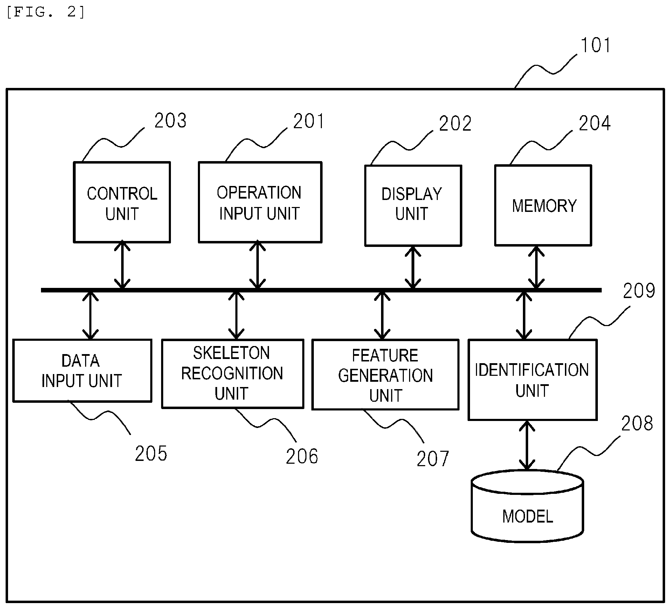

FIG. 2 is a diagram showing an example of a configuration of the motion analysis apparatus 101 according to the present embodiment. The motion analysis apparatus 101 includes an operation input unit 201, a display unit 202, a control unit 203, a memory 204, a data input unit 205, a skeleton recognition unit 206, a feature generation unit 207, a model 208, and an identification unit 209, and the units are connected to one another via a common bus (including a data bus and an address bus).

The operation input unit 201 is a part that receives an instruction from a user (for example, an administrator of the motion analysis apparatus 101) by a mouse operation, a touch panel operation, or the like.

The display unit 202 is a display or the like, and is a part that displays and outputs various types of information. The control unit 203 is a central processing unit (CPU) or the like, and is a part that controls operations of each of the units.

The memory 204 is a part that stores various types of information, such as a memory. The memory 204 temporarily stores, for example, data related to the operation control performed by the control unit 203.

The data input unit 205 is a part that acquires, from the depth camera 102, the depth information or image data that is the result of imaging performed by the depth camera 102 that captures the image of the motion state of the subject 103.

The skeleton recognition unit 206 is a part that recognizes skeleton feature point positions of a human body (subject 103) from the depth information. That is, the skeleton recognition unit 206 is a part that recognizes the skeleton positions of the subject 103 using the depth information acquired by the data input unit 205.

The skeleton recognition unit 206 extracts position information on each of the skeleton positions (information on the skeleton feature point positions) specified from the depth information.

The feature generation unit 207 is a part that extracts indexes (features) designed in advance from temporal transition of the skeleton feature point positions (skeleton feature point coordinates). That is, the feature generation unit 207 is a part that generates the features (for example, a left stride) of the skeleton based on the skeleton positions recognized by the skeleton recognition unit 206.

The model 208 is a part that stores a model in which a correspondence relationship is learned in advance in order to identify walking types based on a condition of the feature. That is, the model 208 is a database that stores model information in which a feature of a motion of a skeleton portion and a walking state corresponding to the feature are defined, and functions as a storage unit.

The identification unit 209 is a part that identifies the walking type of the subject 103 using the feature generated by the feature generation unit 207 and the model stored in the model 208.

In this way, the motion analysis apparatus 101 generates the feature such as a stride and a walking speed based on coordinate changes in skeletons when the subject 103 walks, and identifies the walking type by combining a plurality of features.

Here, the walking type corresponds to a classification of walking that is clinically regarded as abnormal walking such as antalgic gait and steppage gait, a classification of walking for each age such as young people and elderly people, and the like.

These walking types are determined by an expert such as a doctor looking at features of a way of walking, such as the stride and the walking speed.

The motion analysis apparatus 101 quantifies these features based on measurement data of the depth camera 102, and performs identification using a model in which relationships with the walking types are learned by machine learning or the like.

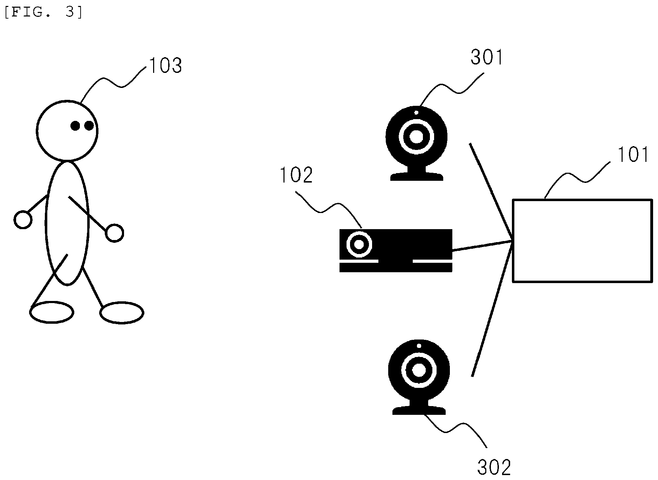

Next, a system that compares depth information obtained from a result of a plurality of devices (skeleton measurement units) capturing images of the same user will be described with reference to FIG. 3.

FIG. 3 is a schematic diagram of the system including the motion analysis apparatus that compares imaging results obtained by the plurality of skeleton measurement units (devices).

As shown in FIG. 3, in addition to the motion analysis apparatus 101 and the depth camera 102 (first device) described with reference to FIG. 1, the system further includes an RGB camera 301 and an RGB camera 302 (second device). The depth camera 102 functions as a first skeleton measurement unit, and the RGB camera 301 and the RGB camera 302 function as a second skeleton measurement unit.

The motion analysis apparatus 101 is connected to the RGB camera 301 and the RGB camera 302 by a USB cable or the like. Further, the motion analysis apparatus 101 may transmit and receive information to and from the RGB camera 301 and the RGB camera 302 via a wireless or wired network.

The RGB camera 301 and the RGB camera 302 are stereo cameras, and can capture the image of the motion state of the user.

A method of estimating a depth by using a stereo camera including the RGB camera 301 and the RGB camera 302 is a method of estimating a depth of an object by using a parallax of two-dimensional images captured from two different directions, which is a known method.

In the system configuration shown in FIG. 3, skeleton coordinate recognition by the depth camera 102 and skeleton coordinate recognition by the RGB camera 301 and the RGB camera 302 are performed for the same walking of the same subject 103, so that an accurate comparison can be made.

In motions such as walking, a movement differs depending on the subject 103, and even for the same subject 103, movements of the skeletons are slightly different every time the subject 103 walks, and therefore, it is desirable to simultaneously measure the same walking of the same subject 103 to perform the comparison and evaluation.

The motion analysis apparatus 101 acquires the depth information from the depth camera 102, and acquires the image data from the RGB camera 301 and the RGB camera 302. The motion analysis apparatus 101 specifies a moving state of the skeletons of the subject 103 based on the depth information acquired from the depth camera 102.

Further, the motion analysis apparatus 101 specifies a moving state (position change) of the skeletons of the subject 103 based on the image data acquired from the RGB camera 301 and the RGB camera 302. Then, the motion analysis apparatus 101 determines whether the respective moving states of the skeletons are similar to each other. Accordingly, the motion analysis apparatus 101 can clarify whether the motion state may be determined using the same model for the results of the imaging performed by two skeleton coordinate acquisition units (the first skeleton measurement unit and the second skeleton measurement unit).

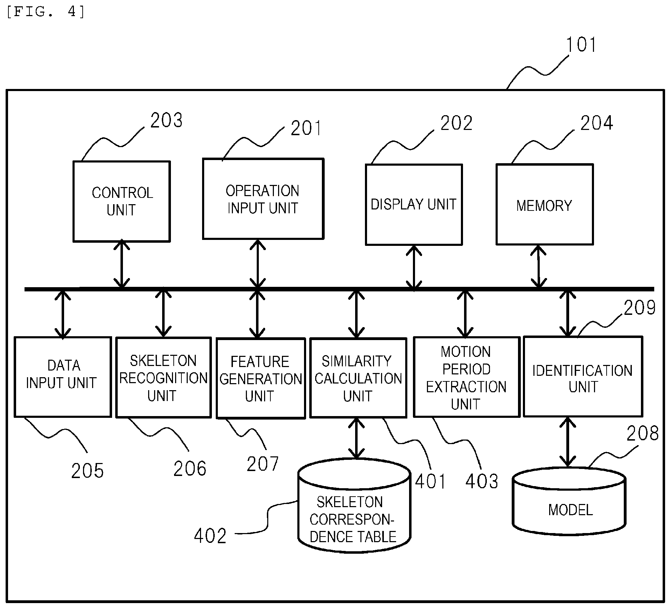

Next, functional details of the motion analysis apparatus 101 will be described with reference to FIG. 4. FIG. 4 is a diagram showing an example of the configuration of the motion analysis apparatus 101 according to the present embodiment.

As shown in FIG. 4, in addition to the configuration of the motion analysis apparatus 101 shown in FIG. 2, the motion analysis apparatus 101 includes a similarity calculation unit 401, a skeleton correspondence table 402, and a motion period extraction unit 403.

The data input unit 205 acquires the image data from the RGB camera 301 and the RGB camera 302. That is, the data input unit 205 acquires a second imaging result that is a result of imaging performed by the second device.

The skeleton recognition unit 206 recognizes the skeleton positions of the subject 103 using the second imaging result (image data acquired from the RGB camera 301 and the RGB camera 302) acquired by the data input unit 205. Specifically, the skeleton recognition unit 206 extracts position information on each of the skeleton positions (information on skeleton feature point positions) specified from the image data acquired from the RGB camera 301 and the RGB camera 302.

The similarity calculation unit 401 compares skeleton feature points recognized for an input from the depth camera 102 with skeleton feature points recognized for an input from the RGB cameras 301 and 302 to calculate similarities.

In addition, the similarity calculation unit 401 outputs a determination result based on the similarities calculated itself to the display unit 202.

In order to compare the skeleton feature points of a first measurement unit (depth camera 102) and a second measurement unit (RGB camera 301 and RGB camera 302), it is necessary to know in advance a correspondence relationship between the skeleton feature points recognized by the first and second measurement units.

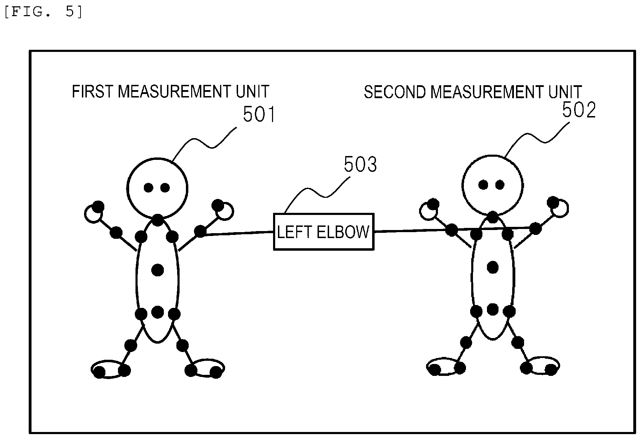

FIG. 5 shows an input screen for allowing the user to designate this correspondence relationship. FIG. 5 is a diagram showing an example of the input screen for inputting the correspondence relationship between the skeleton feature points. In an image 501, positions of the recognized skeleton feature points are superimposed on a subject image acquired by the first measurement unit and displayed by black circles.

Similarly, in an image 502, positions of the recognized skeleton feature points are superimposed on a subject image acquired by the second measurement unit.

The data input unit 205 acquires the image data and the depth data from the depth camera 102, the RGB camera 301, and the RGB camera 302, and the skeleton recognition unit 206 extracts the position information on each of the skeleton positions and then outputs the screen shown in FIG. 5 to the display unit 202.

When the user selects one skeleton feature point from each of the image 501 and the image 502, the skeleton recognition unit 206 associates and stores the skeleton feature points. At this time, a name 503 of the skeleton feature points such as a "left elbow" may be given. All the skeleton feature points are stored in association with each other to form the skeleton correspondence table 402.

As described above, the skeleton recognition unit 206 specifies the correspondence relationships between the skeleton positions of the user recognized from the image data or the depth information of the depth camera 102 acquired by the data input unit 205 and the skeleton positions of the user recognized from the image data of the RGB camera 301 and the RGB camera 302 acquired by the data input unit 205.

Accordingly, the motion analysis apparatus 101 can compare the same or corresponding skeleton portion by associating the skeleton positions specified from the information acquired from both devices.

The skeleton recognition unit 206 can recognize the skeleton positions more accurately by recognizing the skeleton positions according to a user operation on the screen as shown in FIG. 5.

The motion period extraction unit 403 extracts a period from a start of a motion to an end of the motion as a range of data for comparing the skeleton feature points recognized by the skeleton recognition unit 206. In a case of walking, for example, one cycle of the walking from landing of a right foot to next landing of the right foot again is set as a comparison range. In order to extract one walking cycle, for example, a Y-axis coordinate value of a right ankle may be observed, and the start and the end may be determined by regarding a timing at which the Y-axis coordinate value becomes a minimum value as landing.

Specifically, the motion period extraction unit 403 specifies one walking cycle from the landing of the right foot to the landing of the right foot based on changes in the skeleton feature point positions based on the depth information of the depth camera 102 and recognized by the skeleton recognition unit 206. The motion period extraction unit 403 specifies one walking cycle from the landing of the right foot to the landing of the right foot based on changes in the skeleton feature point positions based on the image data of the RGB camera 301 and the RGB camera 302 and recognized by the skeleton recognition unit 206.

In this way, the motion period extraction unit 403 specifies a predetermined motion period of the user based on results recognized by the skeleton recognition unit 206. Further, the motion period extraction unit 403 specifies the walking cycle of the subject 103 based on the results recognized by the skeleton recognition unit 206. Accordingly, the motion analysis apparatus 101 can perform the comparison during the same period of the motion.

The similarity calculation unit 401 calculates the similarity between the change in the skeleton positions of the subject 103 recognized by the skeleton recognition unit 206 using a first imaging result and the change in the skeleton positions of the subject 103 recognized by the skeleton recognition unit 206 using a second imaging result in the motion period specified by the motion period extraction unit 403.

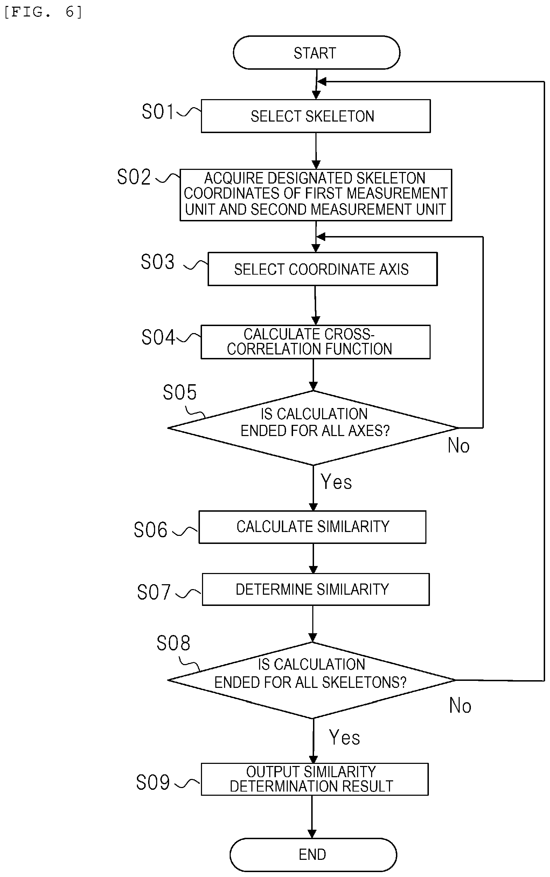

Here, a processing procedure in which the similarity calculation unit 401 calculates the similarity of the skeleton feature points will be described with reference to a flowchart shown in FIG. 6.

FIG. 6 is a flowchart of the processing procedure for calculating the similarity of the skeleton feature points in the similarity calculation unit 401.

In step S01, the similarity calculation unit 401 performs processing of selecting any skeleton. For example, the similarity calculation unit 401 selects one skeleton to be subjected to the calculation of the similarity from a left shoulder, a right shoulder, a left ankle, a right ankle, and the like. Here, it is assumed that the left ankle is selected, and the following processing will be described.

In step S02, the similarity calculation unit 401 acquires coordinate data of the skeleton feature point selected in step S01 from each of the first measurement unit and the second measurement unit. The coordinate data of the skeleton feature point (data of the first measurement unit and the second measurement unit) referred to here is data extracted and outputted by the skeleton recognition unit 206.

A skeleton coordinate is recognized three-dimensionally, and includes data acquired at specific sampling intervals for each of an X axis, a Y axis, and a Z axis. Here, for example, the following processing will be described on an assumption that the X axis is selected.

Step S03 is processing of acquiring data of one specific coordinate axis from the skeleton coordinate data acquired in step S02. Specifically, the similarity calculation unit 401 selects one of the X axis, the Y axis, and the Z axis, and acquires skeleton data of the coordinate axis from the data that is extracted and outputted by the skeleton recognition unit 206 and corresponds to the first measurement unit and the second measurement unit.

Step S04 is processing of calculating a cross-correlation function by using the data of the first measurement unit and the second measurement unit for the axis selected in step S03. Here, the cross-correlation function is calculation used to confirm a similarity between two signals, and is obtained by multiplying a first signal by a second signal shifted by a time t and integrating the first signal and the second signal. The shift time t is plotted on a horizontal axis, and an integral value is plotted on a vertical axis to obtain the cross-correlation function.

Step S05 is processing of determining whether the calculation of the cross-correlation function is ended for all the coordinate axes. When the calculation is not ended, a process returns to step S03, and a new axis is selected. Specifically, the similarity calculation unit 401 selects one of the Y axis and the Z axis except for the X axis for which the cross-correlation function is calculated earlier. When the calculation is ended for all the axes, the process proceeds to step S06.

Step S06 is processing of calculating a similarity of the left ankle by using the cross-correlation function calculated for each of the three axes of the left ankle. The similarity is obtained by adding the three cross-correlation functions on the same time axis and taking a maximum value thereof. When the subject 103 walks toward the depth camera 102, a Z-axis coordinate value of the left ankle changes in a large range, and values that can be taken by coordinate values of the X axis and the Y axis are smaller than that of the Z axis. When the cross-correlation function is calculated and added as it is, an influence of the Z axis increases depending on a magnitude of an original value.

In order to prevent this, it is desirable to normalize a coordinate value obtained by the first measurement unit and a coordinate value obtained by the second measurement unit so as to have a minimum value of 0 and a maximum value of 1, respectively.

Step S07 is processing of determining the similarity calculated in step S06 with a specific threshold. When the similarity is greater than the specific threshold, the similarity calculation unit 401 determines that characteristics of the first measurement unit and the second measurement unit are the same for the left ankle.

Conversely, when the similarity is smaller than the threshold (or equal to or smaller than the threshold), the similarity calculation unit 401 determines that the characteristics of the first measurement unit and the second measurement unit are different from each other. The threshold may be determined in advance by a designer as an initial setting, or may be freely set to a value by the user.

Step S08 is processing of determining whether the similarity calculation is ended for all the skeleton feature points. When the similarity calculation is not ended, the process returns to step S01 and the similarity calculation unit 401 selects a new skeleton feature point. Specifically, the skeleton feature points such as the right ankle are selected except for the left ankle for which the similarity is calculated earlier. When the calculation, performed by the similarity calculation unit 401, for all the skeleton feature points is ended, the process proceeds to step S09. It is not always necessary to calculate the similarity for all the skeletons, and the skeleton feature points to be subjected to the calculation of the similarity may be selected by designation by the user.

Step S09 is processing of presenting a result of the similarity determined for each of the skeleton feature points to the user by outputting the result to the display unit 202.

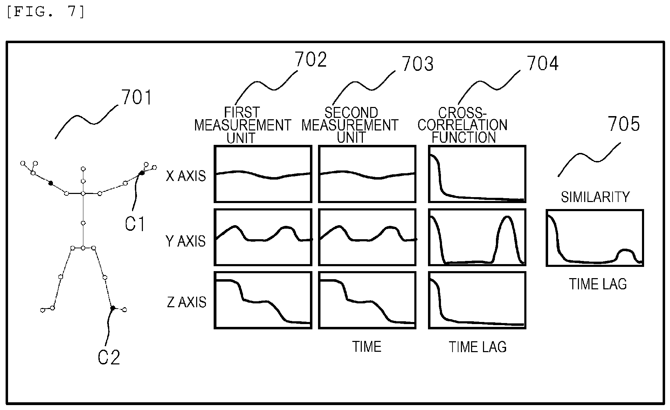

FIG. 7 is a diagram showing an example of a screen configuration for presenting the similarity to the user. A skeleton model 701 (human body portion) schematically showing the skeleton positions of the human body is displayed on a left side of the screen.

In the skeleton model 701, each circle (for example, a skeleton portion C1 and a skeleton portion C2) indicates a position of the skeleton feature point such as a head, a neck, a left shoulder, or a right shoulder. Each circle indicating the skeleton feature point is highlighted and displayed according to the similarity for each of the skeleton feature points calculated in step S06. For example, the similarity calculation unit 401 displays and outputs a left wrist (skeleton portion C1) which is determined to have a small similarity and to have different characteristics between the first measurement unit and the second measurement unit with a black circle to urge the user to pay attention.

Meanwhile, the similarity calculation unit 401 displays and outputs the left wrist (skeleton portion C2), which is determined to have a high similarity and to have the same characteristics between the first measurement unit and the second measurement unit, in a display color (for example, gray) different from black.

In this way, the similarity calculation unit 401 changes the display color according to the similarity and outputs the skeleton portion, thereby allowing the user to easily understand the similarity of each skeleton portion.

Further, the similarity calculation unit 401 may change a color or a size of the circle to be emphasized in a stepwise way according to a magnitude of a value of the similarity of the similarity calculation unit.

When the similarity calculation unit 401 detects that a circle of a specific skeleton feature point (for example, the skeleton portion C2) is selected and instructed as a result of the user operating a pointer or the like, the similarity calculation unit 401 displays measurement data of the skeleton feature point on a right side of the screen.

In a measurement data plot 702 of the first measurement unit, a measurement data plot 703 of the second measurement unit, and a cross-correlation function plot 704, the similarity calculation unit 401 displays the measurement data of the X axis, the Y axis, and the Z axis, respectively.

In addition, the similarity calculation unit 401 also displays a plot 705 of the similarity obtained by adding the cross-correlation functions. In this way, the similarity calculation unit 401 outputs the skeleton model 701 and information indicating the similarity of the skeleton portion (for example, the skeleton portion C2) of the skeleton model 701 as a determination result of the similarity.

By viewing this screen, the user can easily confirm the characteristics of the first measurement unit and the second measurement unit, and an efficiency of a model reconstruction operation can be improved. Here, the similarity is displayed as a graph with respect to a time lag, and the maximum value may be directly displayed as a numerical value.

As a result, the user does not have to read the graph, and can grasp the similarity more simply.

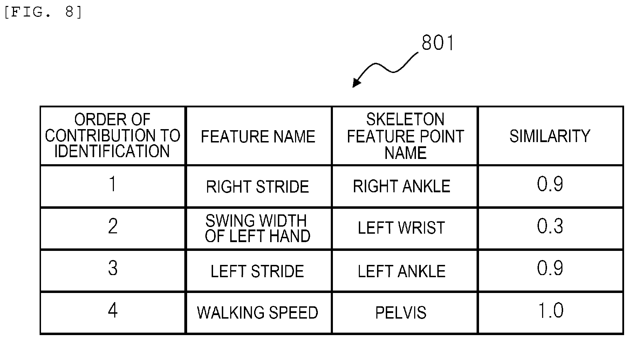

Further, the similarity calculation unit 401 may display and output a table 801 in which correspondences between the skeleton feature points and the similarities are listed as shown in FIG. 8. FIG. 8 is a diagram showing an example of a screen example indicating a table in which the correspondences between the skeleton feature points and the similarities are listed.

The table 801 shown in FIG. 8 includes an "order of contribution to identification" indicating an order of a ratio of contribution when identifying the walking types, a "feature name" indicating a content of the feature, a "skeleton feature point name" indicating a name of the skeleton used to calculate the feature, and a "similarity" indicating the similarity. The order of contribution to identification is determined in advance by the model constructed by the first measurement unit.

The feature contributing to the identification when the model is constructed by the first measurement unit is clarified, and when this feature has a large difference in the characteristics between the first measurement unit and the second measurement unit, the model needs to be reconstructed.

In order to make it easier for the user to confirm the walking type, feature names are listed in descending order of the order of contribution to identification. In addition, skeleton feature point names of original data used for generating the features and the similarities thereof are displayed.

For example, a right stride most contributing to the identification is generated based on data of the right ankle, and a similarity of the right ankle is as high as 0.9. Therefore, it can be determined that the data of the right ankle does not need to be reanalyzed, and there is no problem to use the model constructed by the first measurement unit as it is.

On the other hand, a swing width of a left hand that second contributes to the identification is generated based on data of the left wrist, and a similarity of the left wrist is as low as 0.3. Therefore, the model constructed by the first measurement unit cannot be used as it is for the data of the left wrist, and it is necessary to perform reanalysis. As described above, by presenting the table 801, the user can easily grasp necessity of the reanalysis, and can quickly determine which skeleton feature point data is to be reanalyzed.

According to the above-described embodiment, in the motion analysis apparatus 101, the data input unit 205 acquires the first imaging result and the second imaging result. In the motion analysis apparatus 101, the skeleton recognition unit 206 recognizes the skeleton positions of the subject 103 using the first imaging result acquired by the data input unit 205, and recognizes the skeleton positions of the subject 103 using the second imaging result acquired by the data input unit 205.

The motion period extraction unit 403 extracts the period from the start of the motion to the end of the motion as the range of the data for comparing the skeleton feature points recognized by the skeleton recognition unit 206. The similarity calculation unit 401 compares the skeleton feature points recognized for the input from the depth camera 102 with the skeleton feature points recognized for the input from the RGB cameras 301 and 302 to calculate the similarities, and outputs the determination result based on the similarities.

The motion analysis apparatus 101 can determine whether the model for the first device can be applied to the second device by outputting the result of calculating the similarities of the changes in the skeleton positions of the user based on the imaging results of the different devices as described above, and can efficiently perform data analysis.

### (Second Embodiment)

The first embodiment discloses the example in which when the second measurement unit is introduced for the first time, the efficiency of the model reconstruction operation is improved by visualizing a difference in characteristics, and in an actual environment where measurement is performed, the measurement may be performed only by the second measurement unit and final adjustment of a model may be performed.

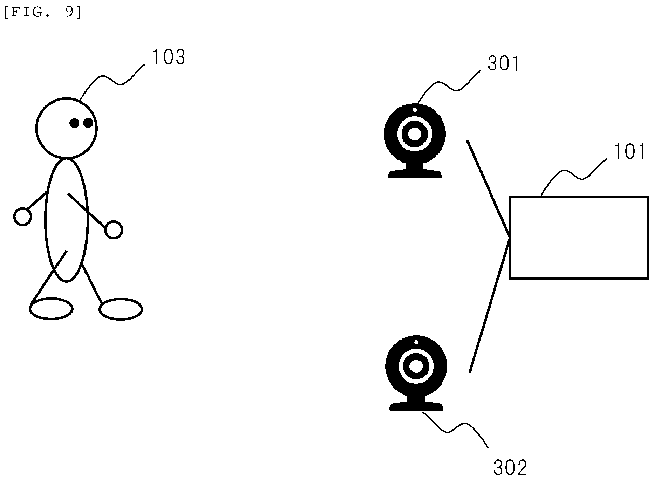

FIG. 9 is a diagram showing a configuration of a system that performs measurement using a motion analysis apparatus according to a second embodiment. The RGB cameras 301 and 302 are connected to the motion analysis apparatus 101, and measure a location where the subject 103 walks toward them. In the present embodiment, it is assumed that the motion analysis apparatus 101 stores in advance a model based on an imaging result acquired from the first device (depth camera 102) described in the first embodiment.

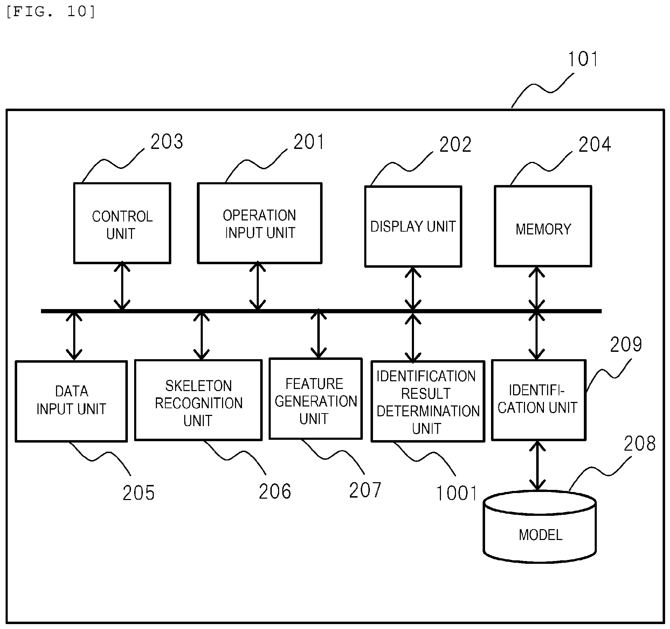

FIG. 10 is a diagram showing an internal configuration of the motion analysis apparatus 101 that performs the final adjustment of the model in a local measurement environment (an environment where the RGB camera 301 and the RGB camera 302 are actually operated).

Blocks denoted by the same reference numerals as those in FIG. 2 have the same functions as those in FIG. 2. An identification result determination unit 1001 is a block that determines whether a result of identification using the model matches a known walking type when walking of a subject that is the known walking type is measured.

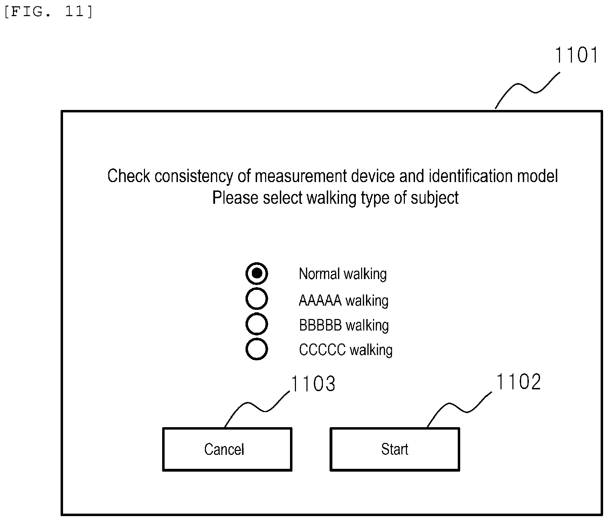

The known walking types are input by a user in advance on a screen as shown in FIG. 11. FIG. 11 is a diagram showing an input screen of walking types (walking type input screen). It is desirable that a walking type input screen 1101 displays the walking types that can be identified by the model as candidates and allows the user to select the walking types.

For example, when the user selects normal walking on the screen, the motion analysis apparatus 101 determines whether a result obtained by measuring the walking and identifying the walking by the model is the normal walking.

That is, when the above selection operation is performed by the operation input unit 201, the data input unit 205 receives an input of the walking type. Further, the data input unit 205 acquires an imaging result for verification (verification imaging result) from the RGB camera 301 and the RGB camera 302 separately from image data from the RGB camera 301 and the RGB camera 302 described in the first embodiment.

The skeleton recognition unit 206 recognizes skeleton positions of the subject 103 using the verification imaging result. Then, the feature generation unit 207 generates features from the skeleton positions of the user based on the verification imaging result recognized by the skeleton recognition unit 206. The identification result determination unit 1001 verifies, using the features generated by the feature generation unit 207 and model information corresponding to the walking type received by the data input unit 205, whether the result corresponds to the walking type received by the data input unit 205. The identification result determination unit 1001 displays and outputs a verification result on the display unit 202.

The walking type input screen 1101 includes a start button 1102 and a cancel button 1103.

When the user selects the start button 1102, the measurement is started, and a determination result is output. When the cancel button 1103 is selected, the screen returns to a previous screen.

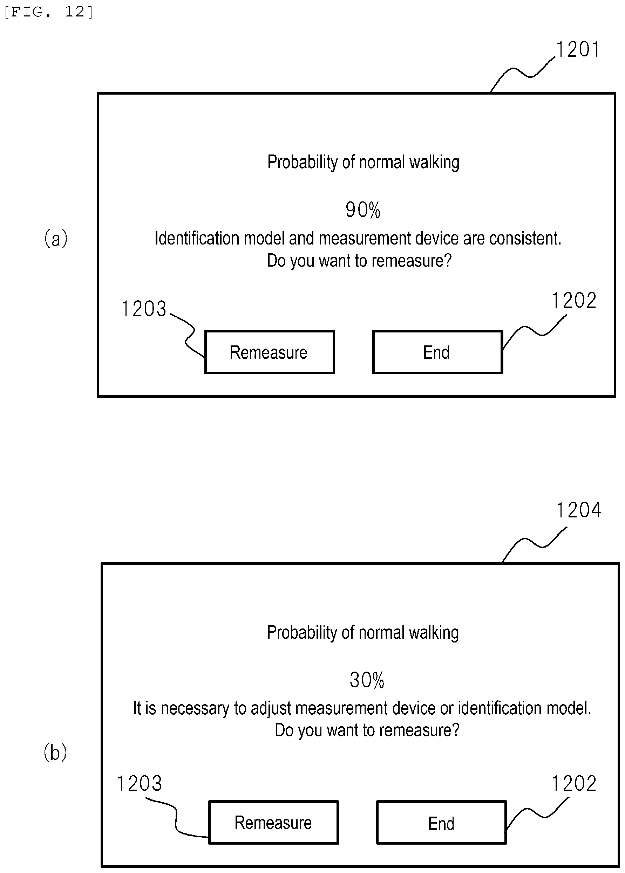

Next, an example a screen of the determination result (verification result) will be described with reference to FIG. 12. FIG. 12 is a diagram showing the example of the screen of the determination result.

When an identification result (determination result) matches the walking type, the user is notified that an identification model and a measurement device are consistent on a screen 1201 as shown in (a) of FIG. 12. Accordingly, the user can determine that it is not necessary to adjust the measurement device and reconstruct the model. An end button 1202 and a re-measurement button 1203 are displayed on the screen 1201. When the user selects the end button 1202, processing of the present embodiment ends, and when the user selects the re-measurement button 1203, processing of walking measurement is performed again.

When the identification result does not match the walking type, a screen 1204 as shown in (b) of FIG. 12 is presented to present the user that it is necessary to adjust the measurement device or the identification model. That is, when it is shown that the verification result by the identification result determination unit 1001 does not correspond to the walking type received by the data input unit 205, the identification result determination unit 1001 outputs information indicating a warning. Accordingly, the user can determine that it is necessary to change an installation environment of the measurement device or to reconstruct the model, and the processing is repeated until the identification result matches the walking type by measuring the walking again.

Further, when the identification result does not match the walking type, coordinate data of the skeleton feature points obtained from the measurement device may be corrected such that the identification result match the walking type. In a correcting method, representative data of the known walking types is stored in advance, and a range in which the coordinate data changes is scaled such that a similarity between the representative data and the coordinate data of the skeleton feature points increases. When the identification results do not match the walking type after the correction, processing of confirming the identification results by further correcting the identification result may be repeated.

### (Third Embodiment)

The first embodiment discloses that the correspondence relationship between the skeleton feature points obtained by the first measurement unit and the second measurement unit is designated by a user, and the corresponding skeleton feature points may be estimated based on the acquired skeleton coordinate data.

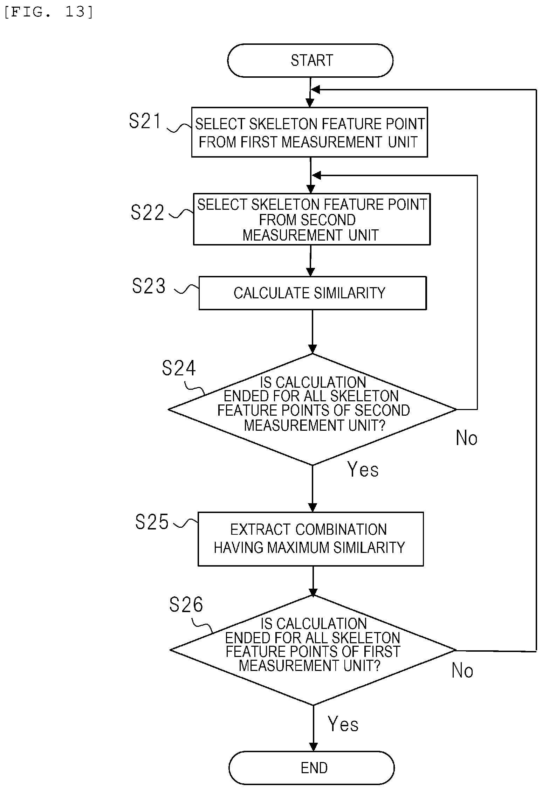

Here, a description will be given with reference to a flowchart shown in FIG. 13. FIG. 13 is a flowchart in which the motion analysis apparatus 101 estimates the correspondence relationship between the skeleton feature points of the first measurement unit and the second measurement unit and generates a skeleton correspondence table.

Step S21 is processing of selecting one skeleton feature point from depth data of the first measurement unit. Here, it is assumed that the skeleton recognition unit 206 selects any skeleton feature point (also referred to as a skeleton feature point 1-1 for convenience) extracted from the depth data.

Step S22 is processing of selecting a skeleton feature point specified based on image data obtained by imaging performed by the second measurement unit from a plurality of directions. Here, it is assumed that the skeleton recognition unit 206 selects any skeleton feature point specified based on the image data obtained by the imaging performed by the second measurement unit from the plurality of directions.

Step S23 is processing of calculating the similarity for a combination of the skeleton feature points selected in steps S21 and S22. The similarity is obtained by calculating cross-correlation functions of an X axis, a Y axis, and a Z axis for two skeleton feature points as described in the first embodiment, and adding the cross-correlation functions to obtain a maximum value of a similarity function.

Step S24 is processing of determining whether calculation of the similarity is ended using all the skeleton feature points of the second measurement unit. When the calculation is not ended, a process returns to step S22, and another skeleton feature point is selected. When the calculation is ended, the process proceeds to step S25.

In step S25, a combination of the skeleton feature points having the maximum value of the similarity is extracted from the similarities comprehensively calculated for all the skeleton feature points extracted from the depth data. For example, when a similarity between the skeleton feature point 1-1 and a skeleton feature point 2-1 is maximum, it is determined that the combination has the correspondence relationship, and the combination is stored in the skeleton correspondence table 402.

In step S26, it is determined whether processing of finding the combination having the correspondence relationship for all the skeleton feature points of the first measurement unit is ended. When the processing is not ended, the process returns to step S21 to select another skeleton feature point 1-2. When the processing is ended, the correspondence relationships are generated for all the skeleton feature points, and the process of the present embodiment is ended.

When the correspondence relationship between the skeleton feature points estimated by this method are displayed on the screen shown in FIG. 5 and the user is caused to perform final confirmation and correction using the correspondence relationship as an initial value, input work of the user is reduced and efficiency is improved.

### (Fourth Embodiment)

The first and third embodiments disclose the case in which types of skeleton feature points obtained by the first measurement unit and the second measurement unit are the same, and when the skeleton feature points of the first measurement unit and the second measurement unit are different from each other, the skeleton feature points having similar coordinate changes may be substituted.

In the present embodiment, a method will be described in which, when the number of types of the skeleton feature points obtained by the second measurement unit is smaller than that of the first measurement unit, the skeleton feature points having the similar coordinate changes are specified and substituted with data thereof.

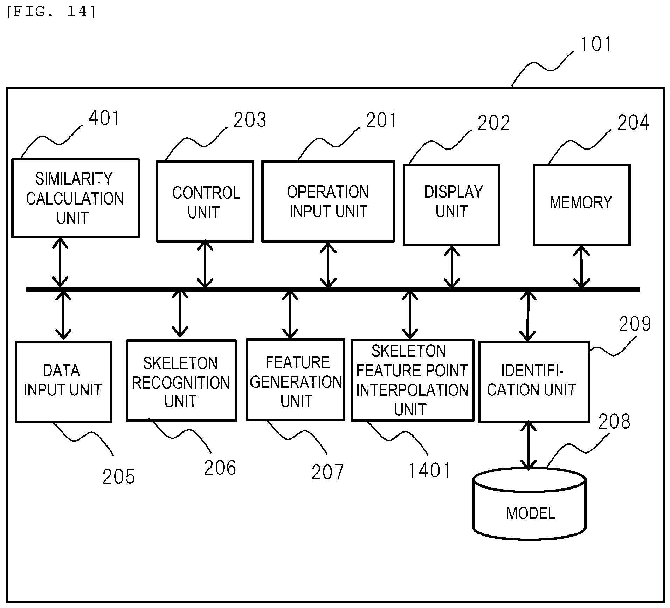

FIG. 14 is a diagram showing a configuration of a motion analysis apparatus according to a fourth embodiment. The motion analysis apparatus 101 includes a skeleton feature point interpolation unit 1401 (skeleton position interpolation unit) in addition to the configuration of the first embodiment. The skeleton feature point interpolation unit 1401 interpolates data of a skeleton feature point that is present in the skeleton feature points recognized by the first measurement unit and is not present in skeleton feature points recognized by the second measurement unit. That is, the skeleton feature point interpolation unit 1401 is a part that specifies, when skeletons of a user recognized by the skeleton recognition unit 206 using a first imaging result are not present in skeletons of the user recognized using a second imaging result, positions corresponding to the positions of the skeletons of the user recognized by the skeleton recognition unit 206 using the first imaging result.

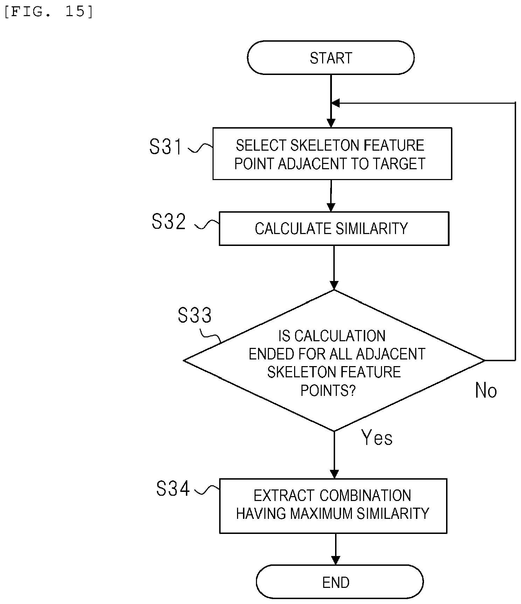

FIG. 15 is a flowchart of specifying a similar skeleton feature point of the first measurement unit with respect to a skeleton feature point (target feature point) of the first measurement unit that is not present in the skeleton feature points of the second measurement unit.

Step S31 is processing of selecting one skeleton feature point of a first measurement method adjacent to the target feature point. Since the adjacent skeleton feature points indicate similar coordinate changes due to characteristics of a human body, when the target feature point is, for example, a right elbow, a right wrist and a right shoulder are served as the adjacent skeleton feature points, and the skeleton feature point interpolation unit 1401 selects one of the right wrist and the right shoulder.

Step S32 is processing of calculating a similarity using the target feature point and the skeleton feature point selected in step S31. The similarity is obtained by calculating cross-correlation functions of an X axis, a Y axis, and a Z axis for two skeleton feature points as described in the first embodiment, and adding the cross-correlation functions to obtain a maximum value of a similarity function.

Step S33 is processing of determining whether calculation of the similarity is ended for all the skeleton feature points adjacent to the target feature point. When the calculation is not ended, a process returns to step S31, and another skeleton feature point is selected. When the calculation is ended, the process proceeds to step S34.

Step S34 is processing of extracting, by the skeleton feature point interpolation unit 1401, a combination having the largest similarity from the similarities calculated for the target feature point. For example, when the target feature point is the right elbow and the similarity to the right wrist is maximum, it is determined that the coordinate change in the right elbow can be substituted by the that of the right wrist, and data of the right elbow is substituted by that of the right wrist in the second measurement unit.

By presenting the user that it is determined that the data of the right elbow can be substituted with that of the right wrist by this method and allowing the user to confirm this substitution, it is possible to prevent unintended substitution by the user.

Instead of substituting with one adjacent skeleton feature point, the data of the target feature point may be generated by interpolation or extrapolation from a plurality of adjacent skeleton feature points. In a case of walking, since an arm often swings while an elbow is extended, when the right elbow is the target feature point, the coordinate change can be obtained by the interpolation from the right wrist and the right shoulder.

Then, when the skeletons of the user recognized by the skeleton recognition unit 206 using the first imaging result are not present in the skeletons of the user recognized using the second imaging result, the similarity calculation unit 401 calculates the similarities between changes in the skeleton positions of the user recognized by the skeleton recognition unit 206 using the first imaging result and changes in skeleton positions at a position that is specified by the skeleton feature point interpolation unit 1401 and corresponds to the skeleton position of the user recognized using the first imaging result.

Accordingly, the motion analysis apparatus 101 can calculate the similarities even if there is no information on the same skeleton position from the first imaging result and the second imaging result.

The invention is not limited to the above-mentioned embodiments, and includes various modifications. For example, the above-described embodiments are described in detail for easy understanding of the invention, and the invention is not necessarily limited to an embodiment including all the configurations described above. A part of a configuration of a certain embodiment can be substituted with a configuration of another embodiment, and can be added to, deleted from, or substituted with the configuration of a certain embodiment by using another configuration. The configurations, functions, processing units, processing methods, or the like may be implemented by hardware by designing a part or all of them with, for example, an integrated circuit. Further, the above-described configurations, functions, and the like may be implemented by software by means of a processor interpreting and executing a program for implementing respective functions. Information such as a program, a table, and a file for implementing each of the functions can be stored in a recording device such as a memory, a hard disk, or a solid state drive (SSD), or in a recording medium such as an IC card, an SD card, or a DVD.

Further, in the above-described embodiments, the walking is taken as an example of the motion to be analyzed, and the invention may be applied to any motion.

### Industrial Applicability

The invention is applicable to a system that processes information acquired from a device capable of capturing an image of a user.

### Reference Signs List

- 101: motion analysis apparatus
- 201: operation input unit
- 202: display unit
- 203: control unit
- 204: memory
- 205: data input unit
- 206: skeleton recognition unit
- 207: feature generation unit
- 208: model
- 401: similarity calculation unit
- 402: skeleton correspondence table
- 403: motion period extraction unit

## Claims

1. An information processing apparatus for processing information acquired from a device capable of capturing an image of a user, the information processing apparatus comprising:
a first imaging result acquisition unit configured to acquire a first imaging result that is a result of imaging performed by a first device configured to capture an image of a motion state of the user;
a second imaging result acquisition unit configured to acquire a second imaging result that is a result of imaging performed by a second device configured to capture an image of the motion state of the user;
a skeleton recognition unit configured to recognize a skeleton position of the user by using the first imaging result acquired by the first imaging result acquisition unit and recognize a skeleton position of the user by using the second imaging result acquired by the second imaging result acquisition unit;
a motion period specifying unit configured to specify a predetermined motion period of the user based on a result obtained by the recognition performed by the skeleton recognition unit;
a similarity calculation unit configured to calculate a similarity between a change in the skeleton position of the user recognized by the skeleton recognition unit using the first imaging result and a change in the skeleton position of the user recognized by the skeleton recognition unit using the second imaging result in the motion period specified by the motion period specifying unit; and
a determination result output unit configured to output a determination result based on the similarity calculated by the similarity calculation unit.

2. The information processing apparatus according to claim 1, wherein
the motion period specifying unit specifies a walking cycle of the user based on the result obtained by the recognition performed by the skeleton recognition unit.

3. The information processing apparatus according to claim 1, wherein
the determination result output unit outputs information indicating that the first device and the second device are similar to each other when the similarity calculated by the similarity calculation unit is greater than a predetermined threshold, and outputs information indicating that characteristics of the first device and the second device are different from each other when the similarity calculated by the similarity calculation unit is equal to or smaller than the predetermined threshold.

4. The information processing apparatus according to claim 1, wherein
the determination result output unit outputs, as the determination result, a human body portion and information indicating a similarity of a skeleton portion of the human body portion.

5. The information processing apparatus according to claim 1, wherein
the skeleton recognition unit further acquires information indicating a skeleton position designated by the user, and recognizes the skeleton position of the user.

6. The information processing apparatus according to claim 1, further comprising:
a storage unit configured to store, for each walking type, model information in which a feature of a motion of a skeleton portion and a walking state corresponding to the feature are defined;
a reception unit configured to receive an input of the walking type;
a verification imaging result acquisition unit configured to acquire a verification imaging result that is a result of imaging performed by the second device for verification;
a feature generation unit configured to generate a feature of the skeleton based on the skeleton position recognized by the skeleton recognition unit;
a verification unit configured to verify, using the feature generated by the feature generation unit and model information corresponding to the walking type received by the reception unit, whether a verification result corresponds to the walking type received by the reception unit; and
a verification result output unit configured to output the verification result by the verification unit, wherein
the skeleton recognition unit recognizes the skeleton position of the user from the verification imaging result acquired by the verification imaging result acquisition unit, and
the feature generation unit generates the feature from the skeleton position of the user based on the verification imaging result recognized by the skeleton recognition unit.

7. The information processing apparatus according to claim 6, wherein
the verification result output unit outputs information indicating a warning when the verification result by the verification unit does not correspond to the walking type received by the reception unit.

8. The information processing apparatus according to claim 1, wherein
the skeleton recognition unit specifies a correspondence relationship between the skeleton position of the user recognized from the first imaging result acquired by the first imaging result acquisition unit and the skeleton position of the user recognized from the second imaging result acquired by the second imaging result acquisition unit.

9. The information processing apparatus according to claim 1, further comprising:
a skeleton position interpolation unit configured to specify a position corresponding to the skeleton position of the user recognized by using the first imaging result when a skeleton of the user recognized by the skeleton recognition unit using the first imaging result is not present in a skeleton of the user recognized by the skeleton recognition unit using the second imaging result, wherein
when the skeleton of the user recognized by the skeleton recognition unit using the first imaging result is not present in the skeleton of the user recognized by the skeleton recognition unit using the second imaging result, the similarity calculation unit calculates a similarity between the change in the skeleton position of the user recognized by the skeleton recognition unit using the first imaging result and a change in a skeleton position at the position that is specified by the skeleton position interpolation unit and corresponds to the skeleton position of the user recognized by using the first imaging result.

10. A determination result output method executed by an information processing apparatus for processing information acquired from a device capable of capturing an image of a user, the determination result output method comprising:
a first imaging result acquisition step of acquiring a first imaging result that is a result of imaging performed by a first device that captures an image of a motion state of the user;
a second imaging result acquisition step of acquiring a second imaging result that is a result of imaging performed by a second device configured to capture an image of the motion state of the user;
a skeleton recognition step of recognizing a skeleton position of the user by using the first imaging result acquired in the first imaging result acquisition step, and recognizing a skeleton position of the user by using the second imaging result acquired in the second imaging result acquisition step;
a motion period specifying step of specifying a predetermined motion period of the user based on a result obtained by the recognition performed in the skeleton recognition step;
a similarity calculation step of calculating a similarity between a change in the skeleton position of the user recognized in the skeleton recognition step using the first imaging result and a change in the skeleton position of the user recognized in the skeleton recognition step using the second imaging result in the motion period specified in the motion period specifying step; and
a determination result output step of outputting a determination result based on the similarity calculated in the similarity calculation step.
